# FASCICULE DE BREVET EUROPEEN

(11) **EP 0 749 952 B1**
(45) Date de publication et mention de la délivrance du brevet: **29.05.2002**
(21) Numéro de dépôt: 96401328.8
(22) Date de dépôt: 19.06.1996
(51) Int. Cl.: C07C 51/56, C07C 57/155

(54) **Procédé de préparation d'anhydride citraconique**
Verfahren zur Herstellung von Citrakonsäureanhydrid
Process for the preparation of citraconic anhydride

(30) Priorité: 21.06.1995 FR 9507395
(43) Date de publication de la demande: 27.12.1996
(60) Demande divisionnaire: 01124485.2
(73) Titulaire: RHODIA CHIMIE, 92408 Courbevoie Cédex (FR)
(72) Inventeur: Alas, Michel, 79500 Melle (FR)
(74) Mandataire: Dubruc, Philippe

(56) Documents cités:
- EP-A- 0 341 112
- EP-A- 0 495 544
- EP-A- 0 665 211
- WO-A-94/21589
- WO-A-95/06026
- BE-A- 702 247
- GB-A- 827 638
- US-A- 2 966 498

## Description

La présente invention concerne un nouveau procédé de préparation de l'anhydride citraconique. Plus précisément, l'invention a trait à un procédé de préparation de l'anhydride citraconique, à partir d'une matière première comprenant de l'acide itaconique.

Dans une variante de l'invention, on prépare l'anhydride citraconique en partant directement du moût de fermentation de l'acide itaconique ou de sous-produits de fabrication de l'acide itaconique.

L'anhydride citraconique est un produit de plus en plus recherché par l'industrie. Depuis plusieurs décennies, il a fait l'objet de nombreuses recherches afin de définir des procédés aussi économiques que possible pour le fabriquer.

Les voies les plus intéressantes consistent à mettre en oeuvre de l'acide itaconique, généralement obtenu par fermentation, à partir de matières premières renouvelables (sucres variés, amidons...), en effectuant une réaction d'anhydrisation couplée à une réaction d'isomérisation.

Le brevet US-A 827 638 mentionne ainsi la préparation à partir d'acide itaconique, en effectuant la réaction de déshydration/isomérisation en discontinu sous pression réduite à des températures comprises entre 155°C et 185°C.

Pour améliorer le rendement de la réaction, il a été proposé d'utiliser un catalyseur pour accélérer la réaction d'isomérisation. Ainsi, par exemple, il est décrit dans le brevet US-A 2 966 498, la fabrication d'anhydride citraconique à partir d'acide itaconique, en utilisant un catalyseur à base de dihydrogéno- phosphate et de sulfate de métaux alcalins.

Ledit procédé est intéressant du fait que l'anhydride citraconique est obtenu en une seule étape mais il souffre de plusieurs inconvénients. La régulation de la température est délicate à assurer. L'élimination de l'eau formée, en faible quantité au cours de la réaction est difficile à réaliser et sa distillation entraîne également de l'anhydride citraconique ce qui conduit à une perte du produit obtenu.

La nature chimique du catalyseur pouvant provoquer divers problèmes à savoir de problèmes de stabilité et de solubilité, on a recherché d'autres systèmes catalytiques performants pouvant être utilisés en quantités aussi faibles que possible pour ne pas provoquer de dégradation et réduire les problèmes technologiques.

Ainsi, Galanti et al ont montré l'intérêt d'utiliser des bases comme catalyseur pour isomériser l'anhydride itaconique en anhydride citraconique [J. Org. Chem. 47, 1572-1574, (1982)]. L'inconvénient de cette synthèse est double, puisqu'il faut fabriquer d'abord l'anhydride itaconique à partir d'acide itaconique. En outre, les amines sont bien connues pour être capables de déclencher des polymérisations violentes de l'anhydride citraconique, ce qui est très gênant au plan industriel pour fabriquer ce produit en toute sécurité.

Par ailleurs, il est mentionné dans EP-A 0 495 544 de préparer l'anhydride citraconique par réaction de l'acide itaconique et de l'anhydride acétique, en présence d'une amine tertiaire ou d'une amine secondaire encombrée ou d'une phosphine ; la réaction étant conduite dans un co-solvant de type hydrocarbure.

Dans WO 94/21589, on a décrit un procédé de préparation d'anhydride citraconique à partir de l'acide itaconique ou citrique en utilisant un catalyseur (amide), en milieu liquide.

Ces deux derniers documents sont muets sur la nature de l'acide itaconique mis en oeuvre.

Afin d'améliorer les rendements et de réduire les risques liés à l'utilisation d'amines, il a été a proposé dans la demande de brevet français n°94 00938, de nouveaux systèmes catalytiques, à savoir des catalyseurs acido-basiques ayant un pKa compris entre 4 et 10.

Poursuivant ses recherches, la demanderesse a perfectionné le procédé de préparation de l'acide itaconique.

L'objet de la présente invention est de faire appel non pas à de l'acide itaconique pur ou semi-pur mais de mettre en oeuvre comme source d'acide itaconique, le moût de fermentation de l'acide itaconique ou des sous-produits de fabrication de l'acide itaconique, notamment des eaux résiduaires.

Plus précisément, le procédé de préparation de l'anhydride citraconique selon l'inveniton est caractérisé par le fait qu'il consiste à mettre en oeuvre une réaction de déshydratation/isomérisation, en continu ou en discontinu , en présence d'un catalyseur acido-basique au moins partiellement organique ayant un pka compris entre 4 et 10 et en introduisant dans le milieu réactionnel maintenu à une température telle que l'acide itaconique présent soit liquide dans les conditions réactionelles, une source comprenant de l'acide itaconique choisie parmi :
□ le moût de fermentation de l'acide itaconique, ayant subi préalablement à la réaction, une opération de séparation du mycélium, si nécessaire un traitement acide, et éventuellement une étape de concentration ou éventuellement une étape d'extraction de l'acide itaconique par un solvant ou un mélange de solvants et une étape de concentration ;
□ les eaux résiduaires sous-produites lors de la purification de l'acide itaconique, ayant éventuellement subi, préalablement à la réaction, une opération d'extraction de l'acide itaconique par un solvant ou un mélange de solvants.

Le procédé de préparation peut être mis en oeuvre en discontinu ou en continu. Dans ce dernier cas, il est très intéressant quand il s'agit de produire de grandes quantités d'anhydride citraconique. La taille du réacteur continu effectuant la même production est en effet beaucoup plus réduite que celle du réacteur l'effectuant en discontinu.

En outre, le fait de faire la réaction en continu est bénéfique au niveau rendement d'où un gain sur les coûts de production, et surtout une constance de la qualité du produit obtenu (réduction de la formation de sous-produits).

Conformément au procédé de l'invention, on part d'une source d'acide itaconique dont la nature est précisée ultérieurement.

La réaction de déshydratation/isomérisation, qui conduit à l'obtention de l'anhydride citraconique à partir de l'acide itaconique, peut être conduite en l'absence de tout solvant ou bien en présence d'un solvant., qui conduit à l'obtention de l'anhydride citraconique,

Ainsi, la matière première de départ est introduite en continu dans le milieu réactionnel sous forme solide ou bien en solution dans l'eau ou dans un solvant organique.

Le choix du solvant est déterminé en fonction de son aptitude à solubiliser l'acide itaconique de départ.

De plus, il doit être inerte dans les conditions réactionnelles.

Il est préférable d'utiliser comme solvant organique, un liquide formant avec l'eau, un azéotrope binaire dont le point d'ébullition est généralement d'au moins 130°C. Ledit solvant organique est choisi de telle façon que l'azéotrope binaire qu'il forme avec l'eau ait un point d'ébullition inférieur:
- au point d'ébullition de l'anhydride citraconique,
- au point d'ébullition de l'azéotrope binaire que l'anhydride citraconique serait susceptible de former, soit avec l'eau, soit avec le solvant organique lui-même.

Enfin, on choisit de préférence, le solvant organique de telle façon qu'il ne forme pas d'azéotrope ternaire avec l'anhydride citraconique et l'eau, afin de limiter les pertes dudit anhydride.

Les solvants organiques pouvant être utilisés dans le cadre du procédé de l'invention ont de préférence un point d'ébullition compris entre 110°C et 200°C, de préférence de 130°C à 170°C.

A titre d'exemples non limitatifs, on peut citer :
- les hydrocarbures aliphatiques et plus particulièrement les paraffines tels que notamment, l'octane, l'isooctane, le nonane, le décane, l'undécane, le tétradécane ; les hydrocarbures aromatiques comme notamment le toluène, les xylènes, l'éthylbenzène, les diéthylbenzènes, les triméthylbenzènes, le cumène, le pseudocumène, les coupes pétrolières constituées de mélange d'alkylbenzènes notamment les coupes de type Solvesso®,
- les hydrocarbures aliphatiques chlorés comme, par exemple, le 1,1,2-trichioroéthane, le pentachloroéthane, le 1-iodo-2-méthylpropane, le 1-chloro-hexane, le 1-chloro-2-éthylhexane ; les hydrocarbures aromatiques chlorés et plus particulièrement, le chlorobenzène, les chlorotoluènes,
- les éthers et plus particulièrement les éthers aliphatiques comme l'éther butylique, l'éther isobutylique, l'éthylhexyléther, le 1 -butoxy-2-méthoxyéthane, le 1,1-diéthoxybutane, l'éther amylique, l'éther isoamylique, le dipropoxyméthane ; les éthers aromatiques comme le phénylpropyléther, l'oxyde de mésityle,
- les composés nitrés tels que le nitropropane, le nitrobenzène,
- les cétones aliphatiques, cycloaliphatiques ou aromatiques de préférence, la méthyléthylcétone, la méthylisobutylcétone, la diisobutylcétone, la méthyl-n-amylcétone, la méthylisoamylcétone, la cyclohexanone, la méthylcyclohexanone, le diacétone alcool.

On peut mettre en oeuvre un mélange de solvants organiques.

Parmi les solvants précités, on choisit préférentiellement les hydrocarbures aromatiques, et plus spécialement le cumène et le pseudocumène.

La concentration de l'acide itaconique dans le mélange réactionnel (acide itaconique + solvant réactionnel) n'est pas critique. Habituellement, l'acide mis en oeuvre représente de 10 à 100 %, de préférence de 10 à 50 % du poids du solvant réactionnel.

Selon une variante particulière de l'invention, on met en oeuvre dans la réaction de déshydratation/isomérisation en présence d'un catalyseur.

De préférence, on met en oeuvre un catalyseur acido-basique au moins partiellement organique ayant un pKa compris entre 4 et 10 et qui fait l'objet de la demande de brevet française n°94 00938.

Le choix du catalyseur permet d'obtenir directement l'anhydride citraconique, à partir de l'acide itaconique.

Un premier impératif qui préside au choix du catalyseur est que ce soit un sel ayant un pKa compris entre 4 et 10, de préférence compris entre 5 et 9 : le pKa étant défini comme le cologarithme de la constante de dissociation de l'acide mesuré, en milieu aqueux, à 25°C.

Une autre caractéristique du catalyseur utilisé dans le procédé de l'invention est qu'il soit fusible ou fondu, dans la masse réactionnelle. Il importe que son point de fusion soit, de préférence, inférieur à 200°C, et plus préférentiellement inférieur ou égal à 180°C.

Comme mentionné précédemment, le catalyseur associe un acide et une base, avec au moins l'un des deux qui est un composé organique.

Le catalyseur peut être un sel résultant de la réaction d'un acide et d'une base, qui peut être préparé extemporanément.

Pour former le sel, on met généralement en oeuvre, les quantités d'acide et de base requises par la stoechiométrie.

Il peut également s'agir d'un sel préparé in situ, par addition d'un acide et d'une base ou bien par addition seulement d'une base, le sel obtenu résultant de la réaction du réactif acide itaconique et de la base.

Le catalyseur résulte donc de la réaction d'un acide minéral ou organique et d'une base.

Comme exemples non limitatifs de tels acides, on peut citer les acides halogénés tels que l'acide chlorhydrique, l'acide bromhydrique, l'acide fluorhydrique ; les oxyacides halogénés ou non tels que l'acide sulfurique, l'acide pyrosulfurique, l'acide perchlorique, l'acide phosphorique ; les acides sulfoniques halogénés ou non, tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Parmi ces acides, on utilisera de préférence l'acide sulfurique, l'acide chlorhydrique, l'acide bromhydrique, l'acide phosphorique, l'acide trifluorométhanesulfonique, l'acide paratoluènesulfonique et l'acide méthanesulfonique.

L'acide itaconique peut également intervenir dans la préparation du catalyseur ou d'autres acides carboxyliques non volatils dans les conditions réactionnelles.

Pour ce qui est de la base, il s'agit d'un composé donneur d'un doublet électronique.

On peut faire appel à des bases azotées primaires, secondaires ou tertiaires et l'on peut citer plus particulièrement :
- l'ammoniac ;
- des amines primaires telles que la n-propylamine, l'isopropylamine, l'isobutylamine, la n-butylamine, la tertiobutylamine, la n-pentylamine, la 2-méthyl butylamine, la 3-méthylbutylamine, la n-hexylamine, la 2-éthylhexylamine, l'aniline, la laurylamine, la cyclohexylamine, la cyclopentylamine, la benzylamine, la guanidine, l'acétamidine, l'éthanolamine, l'éthylènediamine, l'hexaméthylène-diamine, la N-aminoéthylpyrrolidine, la pyrazoline, la N-aminomorpholine, la N-aminopipéridine, la tétraéthylènepentamine ;
- des amines secondaires telles que la dibutylamine, la dipropylamine, la méthylpropylamine, la méthylbutylamine, la méthylisobutylamine, la méthyltertiobutylamine, la méthylbenzylamine, la ditertiobutylamine, la diéthanolamine, la 1-méthylcyclopentylamine, la 1-méthylcyclohexylamine, la dicyclohexylamine, la morpholine, l'imidazole, la pyrrolidine, l'imidazolidine, la pipérazine, l'indole ;
- des amines tertiaires telles que la triéthylamine, la tributylamine, la diméthylaniline, la pyridine, la pyrazine, la triéthanolamine, la tris(3,6-dioxa heptyl)amine, le 1,8-diaza (5,4,0)bicyclo-7-undécène.

Parmi tous les composés azotés précités, on préfère choisir les bases tertiaires azotées hétérocycliques saturées ou insaturées, et préférentiellement, la pyridine ou la pyrazine. On peut également faire appel aux dérivés de substitution (α-picoline, β-picoline).

Un autre groupe de bases convenant à la mise en oeuvre du procédé de l'invention est constitué par les phosphines.

Les trialcoyl- et triarylphosphines sont utilisées de préférence. On peut citer notamment, la triméthylphosphine, la triéthylphosphine, la tri-n-propylphosphine, la tri-n-butylphosphine, la tricyclohexylphosphine, la triphénylphosphine, la tritolylphosphine.

On donne ci-après des exemples de catalyseurs convenant tout à fait bien à la mise en oeuvre de l'invention :
- le tosylate de pyridinium,
- l'itaconate d'ammonium,
- l'itaconate de pyridinium,
- le chlorhydrate de pyridinium,
- le bromhydrate de phosphinium.

La quantité de catalyseur utilisée dans le procédé de l'invention est telle qu'il représente de 0,1 à 30 %, de préférence de 1 à 4 % du poids de l'acide itaconique.

Conformément au procédé de l'invention, on peut introduire le catalyseur sous forme solide, de manière concomitante avec l'acide itaconique sous forme solide.

Le catalyseur étant soluble dans les conditions réactionnelles, il souhaitable de le faire fondre avant son introduction.

Il est également possible de mettre en oeuvre séparément, le catalyseur en solution dans l'eau ou dans un solvant organique tel que ceux précités,

La température à laquelle s'effectue la réaction est telle que l'acide itaconique soit liquide dans le milieu. Elle est d'au moins environ 130°C.

La température est choisie avantageusement dans une gamme de température allant de 130°C à 180°C, de préférence de 150°C à 170°C.

La réaction est conduite avantageusement sous pression atmosphérique.

Dans le cas où le solvant organique présente une température d'ébullition trop basse, il est possible de conduire le procédé de l'invention, sous pression.

Dans le cas inverse où le solvant possède une température d'ébullition trop élevée, on peut conduire le procédé de l'invention sous pression réduite avantageusement comprise entre 100 mm (13300 Pa) et 500 mm (46500 Pa) de mercure.

Selon une variante préférée du procédé de l'invention, on conduit le procédé de l'invention, sous atmosphère contrôlée de gaz inertes. On peut établir une atmosphère de gaz rares, de préférence l'argon mais il est plus économique de faire appel à l'azote.

D'un point de vue pratique, la réaction est aisément mise en oeuvre.

Un premier mode de réalisation consiste à mettre en oeuvre le procédé de l'invention, dans un réacteur agité ou une cascade de réacteurs

On introduit en continu la source d'acide itaconique, sous forme solide ou en solution, dans le milieu réactionnel maintenu à une température telle qu'il soit liquide.

L'alimentation de l'acide itaconique est telle que son temps de séjour dans le milieu réactionnel est de 0,5 à 5 heures.

Comme mentionné précédemment, un catalyseur peut être ajouté soit avec l'acide itaconique, sous forme solide, soit en solution dans l'eau ou un solvant, mais par voie séparée.

Lorsque l'eau formée par la réaction est éliminée par distillation azéotropique, cette élimination est faite de manière continue.

On soutire en continu l'anhydride citraconique produit, par tout moyen connu, notamment par débordement.

Un autre mode de réalisation pratique de l'invention consiste à mettre en oeuvre le procédé de l'invention dans une colonne de distillation.

On introduit en tête d'une colonne à plateaux la source d'acide itaconique et le catalyseur.

Le nombre de plateaux et le volume de chaque plateau doivent être tels que le temps de séjour de l'acide itaconique soit compris entre 0,3 et 3 heures. La colonne est chauffée à la base, de façon à récupérer en tête, l'eau de réaction sous la forme d'un mélange azéotropique avec le solvant. Cette eau est décantée et soutirée en continu. En pied de colonne, on soutire pour maintenir un niveau constant, une solution d'anhydride citraconique dans le solvant.

L'eau de l'azéotrope binaire eau-solvant organique peut être éliminée, soit par passage dudit azéotrope sur un solide adsorbant l'eau tel que par exemple, les tamis moléculaires avant recyclage, soit par décantation.

On récupère l'anhydride citraconique par les moyens classiques utilisés dans ce domaine technique, de préférence, par distillation ou par extraction.

Conformément au procédé de l'invention, on fait appel comme source d'acide itaconique, le moût de fermentation de l'acide itaconique ou des sous-produits de fabrication de l'acide itaconique, notamment des eaux résiduaires.

La rentabilité du procédé est considérablement améliorée. En effet, le prix de l'acide itaconique est déterminant sur le coût de l'anhydride produit.

Comme déjà indiqué, l'acide itaconique est obtenu par fermentation de solutions sucrées grâce à un champignon appartenant souvent à la famille ASPERGILLUS.

Pour isoler l'acide itaconique à partir du moût de fermentation, il est nécessaire d'éliminer le champignon par filtration puis de purifier par les moyens bien connus de l'Homme du métier afin d'obtenir l'acide itaconique pur.

Ces purifications sont compliquées et nécessitent d'importants moyens en équipement pour être menés à bien. En outre, le rendement n'est jamais excellent.

Tout ceci explique pourquoi le prix de l'acide itaconique pur est très élevé et met clairement en évidence l'économie réalisée selon le procédé de l'invention, en partant de résidus de la fabrication de l'acide itaconique, puisque dans ce cas, la matière première est gratuite et que l'on réduit la charge polluante en valorisant un résidu en un produit noble.

Conformément à l'invention, on peut partir du moût de fermentation et non d'acide itaconique pur ou semi pur pour fabriquer l'anhydride citraconique.

L'acide itaconique est obtenu par fermentation aérobie d'un milieu nutritif qui contient une source hydrocarbonée, une source azotée, des oligo-éléments, au moyen d'un micro-organisme appartenant à la souche des Aspergillus productrices d'acide itaconique.

La source hydrocarbonée utilisée peut être d'origine diverse. Il s'agit généralement de carbohydrates dont les plus utilisés incluent les mono- et di-saccharides comme le glucose, saccharose, fructose, les amidons dans la mesure où ils se trouvent sous une forme assimilable par le microorganisme, et les mélasses de betteraves.

On peut également faire appel au glycérol comme source hydrocarbonée conformément à FR-A-2 702 492.

Dans le cas où l'on utilise l'amidon comme source de carbone, il est intéressant d'ajouter une enzyme amylolytique saccharifiante telle que décrit dans EP-A-0 341 112.

Pour la formulation des milieux nutritifs correspondants, on met en oeuvre selon l'invention préférentiellement une concentration en substrat carboné, variant entre 50 et 200 g/l exprimée en poids par volume.

La source d'azote peut notamment être choisie parmi les composés organiques ou minéraux métabolisables, tels que l'extrait soluble de maïs ("com steep liquor") et/ou de soja, l'urée, le sulfate d'ammonium, chlorure d'ammonium, phosphate d'ammonium, nitrate d'ammonium, etc ... et leurs mélanges.

Le milieu contient en outre des sels minéraux tels que sulfates, chlorures, phosphates, de Ca, Mg, Na, K, Fe, Ni, Co, Cu, Mn, Zn ainsi que d'autres additifs usuels tels que les agents de contrôle de pH et/ou des agents anti-moussants.

A titre de micro-organismes utilisables selon l'invention on mentionnera tout particulièrement les espèces Aspergillus terreus et l'Aspergillus itaconicus.

Il s'agit plus particulièrement d'Aspergillus terreus et de préférence de la souche NRRL 1960 identifiée dans le brevet EP-A-341 112.

Le micro-organisme est introduit dans le milieu de fermentation de manière conventionnelle en soi à l'aide d'inoculum ou de cultures intermédiaires.

La fermentation est convenablement réalisée à un pH acide variant entre environ 1,8 et 5 et à une température d'environ 20°C à environ 40°C ; les conditions optimales dépendant de la souche particulière du micro-organisme employé.

Le moût est agité et aéré, à une température choisie dans l'intervalle précitée, pendant 3 à 7 jours.

On récupère un moût de fermentation titrant de 5 à 20 % en poids d'acide itaconique.

Le procédé de l'invention consiste alors :
- à débarrasser du mycélium le moût de fermentation contenant l'acide itaconique,
- à mettre si nécessaire, l'acide itaconique sous forme d'acide libre, par un traitement acide,
- à éventuellement concentrer le moût de fermentation et l'extraire par un solvant approprié,
- à conduire l'étape de déshydratation/isomérisation telle que décrite,
- à récupérer l'anhydride citraconique obtenu.

Dans une première étape, le moût de fermentation est débarrassé du mycélium selon les techniques classiques de séparation solide/liquide, de préférence, par filtration ou par centrifugation.

Lorsqu'un acide organique est produit par fermentation, il est présent au moins partiellement à l'état combiné, en particulier sous forme de sels. Il convient de le libérer par un traitement acide.

A cet effet, on ajoute un acide minéral fort, de préférence, l'acide sulfurique, chlorhydrique, nitrique ou phosphorique.

La quantité d'acide ajouté est telle que le pH soit abaissé à une valeur inférieure ou égale à environ 5, de préférence, entre 1,5 et 5, et encore plus préférentiellement entre 2 et 2,5.

Il est souhaitable d'effectuer une concentration du moût de fermentation.

Pour ce faire, il est concentré par chauffage à une température comprise entre 50°C et 110°C, sous pression atmosphérique ou sous pression réduite avantageusement comprise entre 100 mm (13300 Pa) et 500 mm (46500 Pa) de mercure.

Le moût obtenu présente une concentration en acide itaconique de 20 à 80 % en poids, de préférence, de 30 à 50 % en poids.

On ajoute alors un solvant peu soluble dans l'eau et formant avec elle un azéotrope ainsi, éventuellement, qu'un catalyseur et l'on poursuit la déshydratation/isomérisation.

On peut se référer à la description précédente des exemples de catalyseur préférés, des solvants et des conditions du procédé.

On obtient une solution d'anhydride citraconique dans le solvant.

Cette solution est ensuite distillée pour récupérer le solvant qui est recyclé et l'anhydride citraconique.

Les diverses impuretés ne possédant pas la structure itaconique ou citraconique et qui sont présentes dans le moût de départ, restent après la récupération de l'anhydride citraconique. Elles sont incinérées.

Dans le cas où le moût contenant l'acide itaconique est de mauvaise qualité (c'est-à-dire qu'il contient d'importantes quantités d'impuretés minérales ou organiques comme par exemple des sels et des sucres), pour réduire les problèmes technologiques liés à l'élimination des lourds lors de la distillation finale de l'anhydride citraconique, il est préférable sans que cela soit une obligation absolue, de procéder à une extraction de l'acide itaconique par un solvant ou un mélange de solvants appropriés.

On fait appel aux solvants classiques d'extraction des acides organiques. A titre d'exemples, on peut citer les solvants suivants mis en oeuvre seuls ou en mélanges : les cétones telles que la méthyléthylcétone, la méthylisobutylcétone, la diisobutylcétone, la méthyl-n-amylcétone, la méthyl-isoamylcétone, la cyclohexanone, la méthylcyclohexanone, le diacétone alcool ; les hydrocarbures aliphatiques chlorés tels que le dichlorométhane, le trichlorométhane, le tétrachlorure de carbone, le dichloroéthane ; les éthers-oxydes tels que par exemple l'oxyde de propyle ou d'isopropyle de préférence, en mélange avec une cétone.

Généralement, le volume de solvant organique représente de 0,5 à 4 fois le volume comprenant le composé à extraire.

La couche organique résultante est concentrée comme précédemment pour récupérer le solvant, la déshydratation/isomérisation étant conduite comme indiquée précédemment.

Une autre variante de mise en oeuvre de l'invention consiste à faire la réaction de déshydratation/isomérisation directement dans la couche organique puis à concentrer la solution d'anhydride citraconique obtenue.

C'est cette dernière technique qu'il est préférable de suivre pour fabriquer l'anhydride citraconique à partir des eaux résiduaires sous produites lors de la purification de l'acide itaconique.

Leur composition est évidemment très variable et l'on précise à titre d'exemples qu'elles renferment généralement de 10 à 50 % en poids de matières sèches qui se répartissent ainsi :
- de 5 à 30 % de sources hydrocarbonées ou restes non fermentescibles,
- de 1 à 10 % de sels minéraux,
- de 5 à 20 % de goudrons et lourds divers,
- de 5 à 25 % d'acide itaconique.

Toutes les opérations décrites ci-dessus faisant appel à un moût de fermentation ou de résidus de fabrication comme source d'acide itaconique, peuvent être réalisées soit en discontinu, soit en continu. Si le processus discontinu est très souple et très utilisé pour des productions faibles ou moyennes, il devient beaucoup moins performant quand il s'agit de grosses productions : la mise en oeuvre en continu est alors préférée.

Les exemples qui suivent illustrent l'invention sans toutefois la limiter.

Dans les exemples, les pourcentages donnés sont en poids sauf mention contraire.

### EXEMPLES

### Exemple 1 :

### - Fabrication à partir de moût simplement concentré.

On prélève 2,000 kg de moût débarrassé de mycélium titrant 9,1 % en acide itaconique dosé par chromatographie liquide haute performance.

On concentre jusqu'à 108°C dans la masse en distillant toute l'eau qui vient.

Au liquide obtenu, on ajoute un poids égal de solvant pseudocumène et 5 g de catalyseur (phosphate de pyridinium).

On distille l'eau sous pression atmosphérique jusqu'à ce qu'il n'en vienne plus.

On obtient une solution organique contenant 28,2 g d'anhydride citraconique dosé par chromatographie liquide haute performance.

Le rendement en anhydride citraconique sur l'acide itaconique dosé dans le moût de départ est de 18 %.

### Exemple

### - Utilisation d'un acide fort avant réaction.

On opère comme dans l'exemple 1 en ajoutant au moût 3 % d'acide sulfurique concentré par rapport à l'acide itaconique dosé, les autres opérations étant identiques.

On obtient une solution organique contenant 119 d'anhydride citraconique dosé par chromatographie liquide haute performance.

Le rendement en anhydride citraconique sur l'acide itaconique dosé dans le moût de départ est de 76 %.

### Exemple 3 :

### - Idem exemple 2 avec solvant cyclohexanone.

On prélève 2,000 kg de moût débarrassé de mycélium (titre en acide itaconique 9,1 % par chromatographie liquide haute performance).

On ajoute 3 % d'acide sulfurique par rapport à l'acide itaconique dosé puis l'on concentre jusqu'à 108°C dans la masse en distillant toute l'eau qui vient.

On alimente en 3 heures, le liquide obtenu maintenu à 90-98°C sur une quantité identique de solvant cyclohexanone et 5 g de catalyseur (phosphate de pyridinium).

On distille l'eau sous pression atmosphérique jusqu'à ce qu'il n'en vienne plus.

On obtient une solution organique contenant 117 g d'anhydride citraconique dosé par chromatographie liquide haute performance.

Le rendement en anhydride citraconique sur l'acide itaconique dosé dans le moût de départ est de 75 %.

### Exemple 4 :

### - Fabrication en continu à partir d'acide itaconique pur.

Dans un réacteur agité chauffé de volume 1 m³ équipé d'une colonne à plateaux, on alimente 200 kg/h d'acide itaconique pur fondu, 200 kg/h de pseudocumène et 1 kg/heure de catalyseur paratoluènesulfonate de pyridinium.

On tire en tête l'eau de réaction en recyclant la couche organique.

En pied un mélange pseudocumène/anhydride citraconique est soutiré pour maintenir le niveau constant dans le réacteur.

En régime stabilisé, on dose par chromatographie liquide haute performance 143 kg/h d'anhydride citraconique dans la solution sortant en pied du réacteur ce qui correspond à un rendement de 83 % par rapport à l'acide itaconique alimenté.

### Exemple 5:

### - Idem à exemple 4 avec un solvant plus léger.

On opère comme dans l'exemple 4 en remplaçant le solvant pseudocumène par le xylène. On l'introduit dans le réacteur, de façon à maintenir la température masse dans la fourchette 160°C-170°C.

Le rendement en anhydride citraconique dosé par rapport à l'acide itaconique pur alimenté est de 84 %.

### Exemple 6:

### - Fabrication en continu à partir de résidu.

Le résidu de purification de l'acide itaconique présente la composition suivante :
- 10 % d'acide itaconique,
- 14 % de sucres divers,
- 10 % de lourds divers,
- 5 % de sels minéraux,
- le complément à 100 % étant de l'eau.

Ledit résidu est alimenté à raison de 220 kg/h dans une colonne d'extraction liquide/liquide également alimentée à contre courant par 600 l/h d'un mélange méthyléthylcétone/éther isopropylique de rapport volumique 80/20.

On récupère une solution organique qui est concentrée en continu dans une colonne à distiller alimentée en eau, à la base de laquelle on récupère un mélange eau/acide itaconique à 30 % d'acide itaconique (température 108°C, débit 20,9 kg/h d'acide 100 %).

Cette solution est concentrée en continu sur un évaporateur à film tombant, de façon à obtenir une solution à 60 % d'acide itaconique.

Cette solution (34,8 kg/h) est alimentée en continu dans un réacteur agité (volume 100 litres) équipé pour la distillation.

On y alimente en même temps que 21 kg/h de solvant pseudocumène et 0,8 kg/h de catalyseur (paratoluènesulfonate de pyridinium).

L'eau de réaction et celle amenée par les réactifs sont tirées en tête et l'on soutire en pied une solution d'anhydride citraconique dans le solvant.

Par dosage par chromatographie liquide haute performance, on constate que l'on produit en régime stabilisé 14,4 kg/h d'anhydride citraconique ce qui correspond à un rendement de 76 % par rapport à l'acide dosé dans le résidu de départ.

### Exemple 7 :

### - Idem exemple 6 en partant d'un moût de fermentation.

On opère comme dans l'exemple 6 en remplaçant le résidu de fabrication utilisé comme matière première par un moût débarrassé du mycélium et contenant 9,1% d'acide itaconique dosé par chromatographie liquide haute performance (débit 220 kg/h).

En régime, on obtient 13,4 kg/h d'anhydride citraconique ce qui correspond à un rendement de 78 %.

### Exemple 8 :

### - A partir d'un moût de fermentation concentré.

Un moût de fermentation contenant 10,1 % d'acide itaconique est débarrassé du mycélium, puis concentré sous pression atmosphérique jusqu'à 33 % d'acide (dosage par chromatographie liquide haute performance).

Cette solution est alimentée à raison de 200 kg/h dans une colonne d'extraction liquide/liquide également alimentée à contre courant par 500 l/h d'un mélange méthyléthylcétone/éther isopropylique de rapport volumique 80/20.

On récupère une solution organique qui est concentrée en continu dans une colonne à distiller alimentée en eau, à la base de laquelle on récupère un mélange eau/acide itaconique à 33 % d'acide itaconique (température 109°C, débit 65 kg/h d'acide 100%)

Cette solution est concentrée en continu sur un évaporateur à film tombant de façon à obtenir une solution à 60 % d'acide itaconique. Cette solution (108 kg/h) est alimentée en continu dans un réacteur agité équipé pour la distillation.

On y alimente en même temps que 49 kg/h de solvant pseudocumène et 0,4 kg/h de catalyseur (paratoluènesulfonate de pyridinium).

L'eau de réaction et celle amenée par les réactifs sont tirées en tête et l'on soutire en pied une solution d'anhydride citraconique dans le solvant.

Par dosage par chromatographie liquide haute performance, on constate que l'on produit en régime stabilisé 45,3 kg/h d'anhydride citraconique ce qui correspond à un rendement de 81 % par rapport à l'acide dosé dans le moût de départ.

### Exemple 9:

### - A partir résidu extraction par méthyléthylcétone seule.

On opère comme dans l'exemple 6 en utilisant de la méthyléthylcétone pure au lieu du mélange méthyléthylcétone/éther isopropylique.

Le rendement en anhydride citraconique dosé par chromatographie liquide haute performance est de 48 %.

### Exemple 10:

### - Fabrication à partir de moût sans concentration préalable

Dans une colonne d'extraction liquide/liquide équivalente à 2,9 plateaux théoriques, on alimente en tête 200 kg/h de moût itaconique filtré titrant 9,1 % d'acide itaconique dosé par chromatographie liquide haute performance. Parallèlement, on alimente à la base 112 kg/h d'un mélange cyclohexanone/oxyde d'isopropyle (teneur en oxyde d'isopropyle 10 %).

On récupère en tête une solution organique contenant 18,0 kg d'acide itaconique dosé par chromatographie liquide haute performance.

On ajoute en continu à cette solution 0,2 kg/h de catalyseur (solution aqueuse à 50 % de phosphate de triéthanolamine).

Cette solution est alimentée en tête d'une colonne à plateaux, diamètre 0,2 mètre comportant 5 plateaux à cloches et un bouilleur, ce dernier ayant un volume 25 litres.

Cette colonne est chauffée par un serpentin alimenté en vapeur 12 bar.

Les vapeurs sortant en tête de la colonne sont condensées, le liquide étant décanté (couche aqueuse et couche organique, mélange cyclohexanone et oxyde isopropyle).

On récupère en pied de colonne de façon à maintenir le niveau constant une solution d'anhydride citraconique dans la cyclohexanone à un débit de 23,2 kg/h et titrant 50,0 % d'anhydride citraconique.

Le rendement en anhydride citraconique est de 75 % par rapport à l'acide dosé dans le moût.

## Revendications

1. Procédé de préparation de l'anhydride citraconique, **caractérisé par le fait qu'**il consiste à mettre en oeuvre une réaction de déshydratation/isomérisation, en continu ou en discontinu , en présence d'un catalyseur acido-basique au moins partiellement organique ayant un pka compris entre 4 et 10 et en introduisant dans le milieu réactionnel maintenu à une température telle que l'acide itaconique présent soit liquide dans les conditions réactionelles, une source comprenant de l'acide itaconique choisie parmi :
□ le moût de fermentation de l'acide itaconique, ayant subi préalablement à la réaction, une opération de séparation du mycélium, si nécessaire un traitement acide, et éventuellement une étape de concentration ou éventuellement une étape d'extraction de l'acide itaconique par un solvant ou un mélange de solvants et une étape de concentration ;
□ les eaux résiduaires sous-produites lors de la purification de l'acide itaconique, ayant éventuellement subi, préalablement à la réaction, une opération d'extraction de l'acide itaconique par un solvant ou un mélange de solvants.

2. Procédé selon la revendication 1, **caractérisé par le fait que** le moût de fermentation résulte de la fermentation d'un milieu nutritif comprenant une source hydrocarbonée, une source azotée, des oligo-éléments, au moyen d'un micro-organisme appartenant à la souche des Aspergillus, de préférence Aspergillus terreus et Aspergillus itaconicus.

3. Procédé selon l'une des revendications 1 ou 2, **caractérisé par le fait que** le moût de fermentation subit une opération de séparation du mycélium, par une opération de séparation solide/liquide, de préférence par filtration.

4. Procédé selon l'une des revendications 1 à 3, **caractérisé par le fait que** le moût de fermentation subit un traitement acide par addition d'un acide minéral fort, de préférence, l'acide sulfurique ou chlorhydrique en une quantité telle que le pH soit inférieur ou égal à 5, de préférence entre 1,5 et 5, et, plus préférentiellement, entre 2 et 2,5.

5. Procédé selon l'une des revendications 1 à 4, **caractérisé par le fait que** le moût de fermentation est concentré à une concentration en acide itaconique comprise entre 20 et 80 %, de préférence entre 30 et 50 % en poids.

6. Procédé selon la revendication 1, **caractérisé par le fait que** les eaux résiduaires ayant une concentration en matières sèches de 10 à 50 % en poids présentent la composition suivante :
- de 5 à 30 % de sources hydrocarbonées ou restes non fermentescibles,
- de 1 à 10 % de sels minéraux,
- de 5 à 20 % de goudrons et lourds divers,
- de 5 à 25 % d'acide itaconique.

7. Procédé selon l'une des revendications 1 à 6, **caractérisé par le fait que** l'extraction de l'acide itaconique est effectuée au moyen d'un solvant organique.

8. Procédé selon la revendication précédente, **caractérisé par le fait que** le solvant est choisi parmi : les cétones, de préférence la méthyléthylcétone, la méthylisobutylcétone, la diisobutylcétone, la méthyl-n-amylcétone, la méthylisoamylcétone, la cyclohexanone, la méthylcyclohexanone, le diacétonealcool ; les hydrocarbures aliphatiques chlorés de préférence, la dichlorométhane, le trichlorométhane, le tétrachlorure de carbone, le dichloroéthane ; les éthers-oxydes de préférence, l'oxyde de propyle ou d'isopropyle, préférentiellement en mélange avec une cétone.

9. Procédé selon la revendication 1, **caractérisé par le fait que** le catalyseur acido-basique est un sel ayant un pka compris entre 5 et 9.

10. Procédé selon la revendication 1, **caractérisé par le fait que** le catalyseur a un point de fusion inférieur à 200°C, et de préférence, inférieur ou égal à 180°C.

11. Procédé selon la revendication 1, **caractérisé par le fait que** le catalyseur est un sel résultant de la réaction d'un acide ou d'une base, préparé extemporanément ou in situ.

12. Procédé selon la revendication 1, **caractérisé par le fait que** le catalyseur est un sel préparé in situ par réaction de l'acide itaconique et d'une base.

13. Procédé selon l'une quelconque des revendications 1, 9 à 12, **caractérisé par le fait que** le catalyseur est préparé à partir d'un acide choisi parmi les acides halogénés tels que l'acide chlorhydrique, l'acide bromhydrique, l'acide fluorhydrique ; les oxyacides halogénés ou non tels que l'acide sulfurique, l'acide pyrosulfurique, l'acide perchlorique, l'acide phosphorique ; les acides sulfoniques halogénés ou non, tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalène-sulfoniques et les acides naphtalènedisulfoniques ; les acides carboxyliques, de préférence l'acide itaconique.

14. Procédé selon la revendication 13, **caractérisé par le fait que** l'acide est l'acide chlorhydrique, l'acide bromhydrique, l'acide sulfurique, l'acide trifluorométhanesulfonique, l'acide phosphorique, l'acide paratoluènesulfonique, l'acide phosphorique, l'acide méthanesulfonique ou l'acide itaconique.

15. Procédé selon l'une des revendications 1, 9 à 14, **caractérisé par le fait que** le catalyseur est préparé à partir d'une base choisie parmi :
- l'ammoniac ;
- les amines primaires telles que la n-propylamine, l'isopropylamine, l'isobutylamine, la n-butylamine, la tertiobutylamine, la n-pentylamine, la 2-méthyl butylamine, la 3-méthylbutylamine, la n-hexylamine, la 2-éthylhexylamine, l'aniline, la laurylamine, la cyclohexylamine, la cyclopentylamine, la benzylamine, la guanidine, l'acétamidine, l'éthanolamine, l'éthylènediamine, l'hexaméthylène-diamine, la N-aminoéthylpyrrolidine, la pyrazoline, la N-aminomorpholine, la N-aminopipéridine, la tétraéthylènepentamine ;
- les amines secondaires telles que la dibutylamine, la dipropylamine, la méthylpropylamine, la méthylbutylamine, la méthylisobutylamine, la méthyltertiobutylamine, la méthylbenzylamine, la ditertiobutylamine, la 1-méthylcyclopentylamine, la 1-méthylcyclohexylamine, la diéthanolamine, la dicyclohexylamine, la morpholine, l'imidazole, la pyrrolidine, l'imidazolidine, la pipérazine, l'indole ;
- les amines tertiaires telles que la triéthylamine, la tributylamine, la diméthylaniline, la pyridine, la pyrazine, la triéthanolamine, la tris(3,6-dioxa heptyl)amine, le 1,8-diaza (5,4,0)bicyclo-7-undécène.
- les trialcoyl- et triarylphosphines, de préférence notamment, la triméthylphosphine, la triéthylphosphine, la tri-n-propylphosphine, la tri-n-butylphosphine, la tricyclohexylphosphine, la triphénylphosphine, la tritolyl-phosphine.

16. Procédé selon la revendication 15, **caractérisé par le fait que** la base est une base azotée hétérocyclique, saturée ou insaturée, éventuellement substituée, de préférence, la pyridine ou la pyrazine.

17. Procédé selon l'une des revendications 1, 9 à 16, **caractérisé par le fait que** le catalyseur est choisi parmi :
- le tosylate de pyridinium,
- l'itaconate d'ammonium,
- l'itaconate de pyridinium,
- le chlorhydrate de pyridinium,
- le bromhydrate de phosphinium.

18. Procédé selon l'une des revendications 1 à 17, **caractérisé par le fait que** la quantité de catalyseur représente de 0,1 à 30 %, de préférence de 0,5 à 4 % du poids de l'acide itaconique.

19. Procédé selon l'une des revendications 1 à 18, **caractérisé par le fait que** la réaction est conduite dans un solvant organique ayant un point d'ébullition compris entre 110°C et 200°C, de préférence de 130°C à 170°C.

20. Procédé selon la revendication 19, **caractérisé par le fait que** le solvant organique est choisi parmi :
- les hydrocarbures aliphatiques et plus particulièrement les paraffines tels que notamment, l'octane, l'isooctane, le nonane, le décane, l'undécane, le tétradécane ; les hydrocarbures aromatiques comme notamment le toluène, les xylènes, l'éthylbenzène, les diéthylbenzènes, les triméthylbenzènes, le cumène, le pseudocumène, les coupes pétrolières constituées de mélange d'alkylbenzènes notamment les coupes de type Solvesso®,
- les hydrocarbures aliphatiques chlorés comme, par exemple, le 1,1,2-trichloroéthane, le pentachloroéthane, le 1-iodo-2-méthylpropane, le 1-chloro-hexane, le 1-chloro-2-éthylhexane ; les hydrocarbures aromatiques chlorés et plus particulièrement, le chlorobenzène, les chlorotoluènes,
- les éthers et plus particulièrement les éthers aliphatiques comme l'éther butylique, l'éther isobutylique, l'éthylhexyléther, le 1-butoxy-2-méthoxyéthane, le 1,1-diéthoxybutane, l'éther amylique, l'éther isoamylique, le dipropoxyméthane ; les éthers aromatiques comme le phénylpropyléther, l'oxyde de mésityle,
- les composés nitrés tels que le nitropropane, le nitrobenzène.
- les cétones aliphatiques, cycloaliphatiques ou aromatiques de préférence, la méthyléthylcétone, la méthylisobutylcétone, la diisobutylcétone, la méthyl-n-amylcétone, la méthylisoamylcétone, la cyclohexanone, la méthylcyclohexanone, le diacétone alcool.

21. Procédé selon la revendication 20, **caractérisé par le fait que** le solvant organique est un hydrocarbure aromatique, de préférence, le cumène ou le pseudocumène.

22. Procédé selon l'une des revendications 1 à 21, **caractérisé par le fait que** la température de la réaction de déshydratation/isomérisation est d'au moins 130°C, de préférence entre 130°C et 180°C et encore plus préférentiellement, entre 150°C et 170°C.

## Patentansprüche

1. Verfahren zur Herstellung von Citraconsäureanhydrid, **dadurch gekennzeichnet, dass** es darin besteht, kontinuierlich oder diskontinuierlich eine Reaktion zur Wasserabspaltung/Isomerisierung durchzuführen in Gegenwart eines Säure-Base-Katalysators mit einem pKa zwischen 4 und 10, der wenigstens teilweise organisch ist, und indem man dem Reaktionsmedium, das auf einer solchen Temperatur gehalten wird, dass die vorhandene Itaconsäure unter den Reaktionsbedingungen flüssig ist, eine Itaconsäure umfassende Quelle zufügt, die ausgewählt ist unter:
° dem Fermentationsmedium der Itaconsäure, das vor der Reaktion einem Arbeitsschritt zur Abtrennung des Myzels, wenn notwendig einer sauren Behandlung und gegebenenfalls einem Schritt zur Konzentration oder gegebenenfalls einem Schritt zur Extraktion der Itaconsäure durch ein Lösungsmittel oder ein Lösungsmittelgemisch und einem Schritt zur Konzentration unterzogen wurde;
° den Abwässern, die bei der Reinigung der Itaconsäure a!s Nebenprodukte anfallen, die gegebenenfalls vor der Reaktion einem Arbeitsschritt zur Extraktion der ltaconsäure durch ein Lösungsmittel oder ein Lösungsmittelgemisch unterzogen wurden.

2. Verfahren gemäß Anspruch 1, **dadurch gekennzeichnet, dass** das Fermentationsmedium aus der Fermentation eines Nährmediums, das eine Kohlenhydratquelle, eine Stickstoffquelle, Spurenelemente umfasst, mit einem Mikroorganismus stammt, der dem Stamm der Aspergillus angehört, vorzugsweise Aspergillus terreus und Aspergillus itaconicus.

3. Verfahren gemäß einem der Ansprüche 1 oder 2, **dadurch gekennzeichnet, dass** das Fermentationsmedium einem Arbeitsschritt zur Abtrennung des Myzels durch einen Arbeitsschritt zur Fest/Flüssig-Trennung, vorzugsweise durch Filtration, unterzogen wird.

4. Verfahren gemäß einem der Ansprüche 1 bis 3, **dadurch gekennzeichnet, dass** das Fermentationsmedium einer sauren Behandlung durch Zugabe einer starken mineralischen Säure, vorzugsweise Schwefelsäure oder Salzsäure, in einer solchen Menge unterzogen wird, dass der pH niedriger oder gleich 5, vorzugsweise zwischen 1,5 und 5 und stärker bevorzugt zwischen 2 und 2,5 ist.

5. Verfahren gemäß einem der Ansprüche 1 bis 4, **dadurch gekennzeichnet, dass** das Fermentationsmedium auf eine Itaconsäurekonzentration zwischen 20 und 80 Gew.-%, vorzugsweise zwischen 30 und 50 Gew.-% konzentriert wird.

6. Verfahren gemäß Anspruch 1, **dadurch gekennzeichnet, dass** die Abwässer mit einer Konzentration an Feststoffen von 10 bis 50 Gew.-% die folgende Zusammensetzung aufweisen:
- 5 bis 30% Kohlenhydratquellen oder nicht fermentierbare Reste,
- 1 bis 10% Mineralsalze,
- 5 bis 20% Teere und verschiedene hochsiedende Stoffe,
- 5 bis 25% Itaconsäure.

7. Verfahren gemäß einem der Ansprüche 1 bis 6, **dadurch gekennzeichnet, dass** die Extraktion der Itaconsäure mit einem organischen Lösungsmittel ausgeführt wird.

8. Verfahren gemäß dem vorhergehenden Anspruch, **dadurch gekennzeichnet, dass** das Lösungsmittel ausgewählt ist unter: den Ketonen, vorzugsweise Methylethylketon, Methylisobutylketon, Diisobutylketon, Methyl-n-amylketon, Methylisoamylketon, Cyclohexanon, Methylcyclohexanon, Diacetonalkohol; den chlorierten aliphatischen Kohlenwasserstoffen, vorzugsweise Dichlormethan, Trichlormethan, Tetrachlorkohlenstoff, Dichlorethan; den Etheroxiden, vorzugsweise Propyl- oder Isopropyloxid, bevorzugt im Gemisch mit einem Keton.

9. Verfahren gemäß Anspruch 1, **dadurch gekennzeichnet, dass** der Säure-Base-Katalysator ein Salz mit einem pKa zwischen 5 und 9 ist.

10. Verfahren gemäß Anspruch 1, **dadurch gekennzeichnet, dass** der Katalysator einen Schmelzpunkt niedriger als 200°C und vorzugsweise niedriger oder gleich 180°C hat.

11. Verfahren gemäß Anspruch 1, **dadurch gekennzeichnet, dass** der Katalysator ein Salz ist, das aus der Reaktion einer Säure oder einer Base stammt, das frisch oder in situ hergestellt wird.

12. Verfahren gemäß Anspruch 1, **dadurch gekennzeichnet, dass** der Katalysator ein in situ durch Reaktion von Itaconsäure und einer Base hergestelltes Salz ist.

13. Verfahren gemäß einem der Ansprüche 1, 9 bis 12, **dadurch gekennzeichnet, dass** der Katalysator aus einer Säure hergestellt wird, die ausgewählt ist unter den halogenhaltigen Säuren, wie Salzsäure, Bromwasserstoffsäure, Fluorwasserstoffsäure; den halogenhaltigen oder nicht halogenhaltigen Oxosäuren, wie Schwefelsäure, Pyroschwefelsäure, Perchlorsäure, Phosphorsäure; den halogen-haltigen oder nicht halogenhaltigen Sulfonsäuren, wie Fluorsulfonsäure, Chlorsulfonsäure oder Trifluormethansulfonsäure, Methansulfonsäure, Ethansulfonsäure, Ethandisulfonsäure, Benzensulfonsäure, die Benzendisulfonsäuren, die Toluensulfonsäuren, die Naphthalensulfonsäuren und die Naphthalendisulfonsäuren; den Carbonsäuren, vorzugsweise Itaconsäure.

14. Verfahren gemäß Anspruch 13, **dadurch gekennzeichnet, dass** die Säure Salzsäure, Bromwasserstoffsäure, Schwefelsäure, Trifluormethansulfonsäure, Phosphorsäure, para-Toluensulfonsäure, Phosphorsäure, Methansulfonsäure oder Itaconsäure ist.

15. Verfahren gemäß einem der Ansprüche 1, 9 bis 14, **dadurch gekennzeichnet, dass** der Katalysator aus einer Base hergestellt wird, die ausgewählt ist unter:
- Ammoniak;
- den primären Aminen, wie n-Propylamin, Isopropylamin, Isobutylamin, n-Butylamin, tert-Butylamin, n-Pentylamin, 2-Methylbutylamin, 3-Methylbutylamin, n-Hexylamin, 2-Ethylhexylamin, Anilin, Laurylamin, Cyclohexylamin, Cyclopentylamin, Benzylamin, Guanidin, Acetamidin, Ethanolamin, Ethylendiamin, Hexamethylendiamin, N-Aminoethylpyrrolidin, Pyrazolin, N-Aminomorpholin, N-Aminopiperidin, Tetraethylenpentamin;
- den sekundären Aminen, wie Dibutylamin, Dipropylamin, Methylpropylamin, Methylbutylamin, Methylisobutylamin, Methyl-tert-butylamin, Methylbenzylamin, Di-tert-butylamin, 1-Methylcyclopentylamin, 1-Methylcyclohexylamin, Diethanolamin, Dicyclohexylamin, Morpholin, Imidazol, Pyrrolidin, Imidazolidin, Piperazin, Indol;
- den tertiären Aminen, wie Triethylamin, Tributylamin, Dimethylanilin, Pyridin, Pyrazin, Triethanolamin, Tris(3,5-dioxaheptyl)amin, 1,8-Diaza[5.4.0]bicyclo-7-undecen;
- den Trialkyl- und Triarylphosphinen, vorzugsweise insbesondere Trimethylphosphin, Triethylphosphin, Tri-n-propylphosphin, Tri-n-butylphosphin, Tricyclohexylphosphin, Triphenylphosphin, Tritolyl-phosphin.

16. Verfahren gemäß Anspruch 15, **dadurch gekennzeichnet, dass** die Base eine gegebenenfalls substituierte, gesättigte oder ungesättigte, heterocyclische stickstoffhaltige Base ist, vorzugsweise Pyridin oder Pyrazin.

17. Verfahren gemäß einem der Ansprüche 1, 9 bis 16, **dadurch gekennzeichnet, dass** der Katalysator ausgewählt ist unter:
- Pyridiniumtosylat,
- Ammoniumitaconat,
- Pyridiniumitaconat,
- Pyridin-hydrochlorid,
- Phosphin-hydrobromid.

18. Verfahren gemäß einem der Ansprüche 1 bis 17, **dadurch gekennzeichnet, dass** die Menge an Katalysator 0,1 bis 30%,vorzugsweise 0,5 bis 4% des Gewichts der Itaconsäure darstellt.

19. Verfahren gemäß einem der Ansprüche 1 bis 18, **dadurch gekennzeichnet, dass** die Reaktion in einem organischen Lösungsmittel mit einem Siedepunkt zwischen 110°C und 200°C, vorzugsweise von 130°C bis 170°C ausgeführt wird.

20. Verfahren gemäß Anspruch 19, **dadurch gekennzeichnet, dass** das organische Lösungsmittel ausgewählt ist unter:
- den aliphatischen Kohlenwasserstoffen und spezieller den Paraffinen, wie insbesondere Octan, Isooctan, Nonan, Decan, Undecan, Tetradecan; den aromatischen Kohlenwasserstoffen, wie insbesondere Toluen, die Xylene, Ethylbenzen, die Diethylbenzene, die Trimethylbenzene, Cumol, Pseudocumol, die Erdölfraktionen, die aus Gemisch von Alkylbenzenen bestehen, insbesondere die Fraktionen vom Typ Solvesso®,
- den chlorierten aliphatischen Kohlenwasserstoffen, wie beispielsweise 1,1,2-Trichlorethan, Pentachlorethan, 1-lod-2-methylpropan, 1-Chlorhexan, 1-Chlor-2-ethylhexan; den chlorierten aromatischen Kohlenwasserstoffen und spezieller Chlorbenzen, die Chlortoluene,
- den Ethern und spezieller den aliphatischen Ethern, wie Butylether, Iso-butylether, Ethylhexylether, 1-Butoxy-2-methoxyethan, 1,1-Diethoxybutan, Amylether, Isoamylether, Dipropoxymethan; den aromatischen Ethern, wie Phenylpropylether, Mesityloxid,
- den nitrierten Verbindungen, wie Nitropropan, Nitrobenzen,
- den aliphatischen, cycloaliphatischen oder aromatischen Ketonen, vorzugsweise Methylethylketon, Methylisobutylketon, Diisobutylketon, Methyl-n-amylketon, Methylisoamylketon, Cyclohexanon, Methylcyclohexanon, Diacetonalkohol.

21. Verfahren gemäß Anspruch 20, **dadurch gekennzeichnet, dass** das organische Lösungsmittel ein aromatischer Kohlenwasserstoff ist, vorzugsweise Cumol oder Pseudocumol.

22. Verfahren gemäß einem der Ansprüche 1 bis 21, **dadurch gekennzeichnet, dass** die Temperatur der Reaktion zur Wasserabspaltung/Isomerisierung wenigstens 130°C beträgt, vorzugsweise zwischen 130°C und 180°C und noch stärker bevorzugt zwischen 150°C und 170°C liegt.

## Claims

1. Process for preparing citraconic anhydride, **characterized in that** it consists in carrying out a continuous or batchwise dehydration/isomerization reaction, in the presence of an at least partially organic acid-based catalyst with a pKa of between 4 and 10, by introducing into the reaction medium, maintained at a temperature such that the itaconic acid present is liquid under the reaction conditions, a source comprising itaconic acid chosen from:
□ the fermentation broth of itaconic acid which has, prior to the reaction, undergone an operation to separate out the mycelium, if necessary an acidic treatment, and optionally a concentration step or optionally a step of extraction of the itaconic acid with a solvent or a mixture of solvents and a concentration step;
□ the residual waters obtained as by-products of the purification of the itaconic acid, which have, prior to the reaction, optionally undergone an operation of extraction of the itaconic acid with a solvent or a mixture of solvents.

2. Process according to claim 1, **characterized in that** the fermentation broth results from the fermentation of a nutrient medium comprising a hydrocarbon source, a nitrogen source and trace elements, using a microorganism belonging to the strain of Aspergillus, preferably Aspergillus terreus and Aspergillus itaconicus.

3. Process according to either of claims 1 and 2, **characterized, in that** the fermentation broth undergoes a mycelium separation operation, by a solid/liquid separation operation, preferably by filtration.

4. Process according to one of claims 1 to 3, **characterized in that** the fermentation broth undergoes an acidic treatment by addition of a strong inorganic acid, preferably sulphuric or hydrochloric acid, in an amount such that the pH is below or equal to 5, preferably between 1.5 and 5 and even more preferably between 2 and 2.5.

5. Process according to one of claims 1 to 4, **characterized in that** the fermentation broth is concentrated to an itaconic acid concentration of between 20 and 80 %, preferably of between 30 and 50 %, by weight.

6. Process according to claim 1, **characterized in that** the residual waters having a solids concentration of from 10 to 50 % by weight have the following composition:
- from 5 to 30 % of non-fermentable residues or hydrocarbon sources,
- from 1 to 10 % of inorganic salts,
- from 5 to 20 % of tars and various heavy fractions,
- from 5 to 25 % of itaconic acid.

7. Process according to one of claims 1 to 6, **characterized in that** the extraction of the itaconic acid is carried out using an organic solvent.

8. Process according to the preceding claim, **characterized in that** the solvent is chosen from: ketones, preferably methyl ethyl ketone, methyl isobutyl ketone, diisobutyl ketone, methyl n-amyl ketone, methyl isoamyl ketone, cyclohexanone, methylcyclohexanone and diacetone alcohol; preferably chlorinated aliphatic hydrocarbons, dichloromethane, trichloromethane, carbon tetrachloride and dichloroethane; preferably oxide ethers, propyl ether or isopropyl ether, preferably as a mixture with a ketone.

9. Process according to claim 1, **characterized in that** the acid-base catalyst is a salt having a pKa of between 5 and 9.

10. Process according to claim 1, **characterized in that** the catalyst has a melting point of below 200°C and preferably of below or equal to 180°C.

11. Process according claim 1, **characterized in that** the catalyst is a salt resulting from the reaction of an acid or a base, prepared at the time of use or in situ.

12. Process according to claim 1, **characterized in that** the catalyst is a salt prepared in situ by reaction of itaconic acid and a base.

13. Process according to any one of claims 1, 9 to 12, **characterized in that** the catalyst is prepared from an acid chosen from halogenated acids such as hydrochloric acid, hydrobromic acid and hydrofluoric acid; oxyacids which may or may not be halogenated, such as sulphuric acid, pyrosulphuric acid, perchloric acid and phosphoric acid; sulphonic acids which may or may not be halogenated, such as fluorosulphonic acid, chlorosulphonic acid, trifluoromethanesulphonic acid, methanesulphonic acid, ethanesulphonic acid, ethanedisulphonic acid, benzenesulphonic acid, benzenedisulphonic acids, toluenesulphonic acids, naphthalenesulphonic acids and naphthalenedisulphonic acids; carboxylic acids, preferably itaconic acid.

14. Process according to claim 13, **characterized in that** the acid is hydrochloric acid, hydrobromic acid, sulphuric acid, trifluoromethanesulphonic acid, phosphoric acid, para-toluenesulphonic acid, phosphoric acid, methanesulphonic acid or itaconic acid.

15. Process according to one of claims 1, 9 to 14, **characterized in that** the catalyst is prepared from a base chosen from:
- ammonia;
- primary amines such as n-propylamine, isopropylamine, isobutylamine, n-butylamine, tert-butylamine, n-pentylamine, 2-methylbutylamine, 3-methylbutylamine, n-hexylamine, 2-ethylhexylamine, aniline, laurylainine, cyclohexylamine, cyclopentylamine, benzylamine, guanidine, acetamidine, ethanolamine, ethylenediamine, hexamethylenediamine, N-aminoethylpyrrolidine, pyrazoline, N-aminomorpholine, N-aminopiperidine and tetraethylenepentamine;
- secondary amines such as dibutylamine, dipropylamine, methylpropylamine, methylbutylamine, methylisobutylamine, methyl-tert-butylamine, methylbenzylamine, di-tert-butylamine, 1-methyl-cyclopentylamine, 1-methylcyclohexylamine, diethanolamine, dicyclohexylamine, morpholine, imidazole, pyrrolidine, imidazolidine, piperazine and indole;
- tertiary amines such as triethylamine, tributylamine, dimethylaniline, pyridine, pyrazine, triethanolamine, tris(3,6-dioxaheptyl)amine and 1,8-diaza(5.4.0)bicyclo-7-undecene;
- trialkyl- and triarylphosphines, preferably, in particular, trimethylphosphine, triethylphosphine, tri-n-propylphosphine, tri-n-butylphosphine, tricyclohexylphosphine, triphenylphosphine and tritolylphosphine.

16. Process according to claim 15, **characterized in that** the base is a saturated or unsaturated heterocyclic nitrogen-containing base which is optionally substituted, preferably pyridine or pyrazine.

17. Process according to one of claims 1, 9 to 16, **characterized in that** the catalyst is chosen from:
- pyridinium tosylate,
- ammonium itaconate,
- pyridinium itaconate,
- pyridinium hydrochloride,
- phosphinium hydrobromide

18. Process according to one of claims 1 to 17, **characterized in that** the amount of catalyst represents from 0.1 to 30 %, preferably from 0.5 to 4 % of the weight of the itaconic acid.

19. Process according to one of claims 1 to 18, **characterized in that** the reaction is performed in an organic solvent having a boiling point of between 110°C and 200°C, preferably of from 130°C to 170°C.

20. Process according to claim 19, **characterized in that** the organic solvent is chosen from:
- aliphatic hydrocarbons and more particularly paraffins such as, in particular, octane, isooctane, nonane, decane, undecane and tetradecane; aromatic hydrocarbons such as, in particular, toluene, xylenes, ethylbenzene, diethylbenzenes, trimethylbenzenes, cumene, pseudocumene and petroleum fractions consisting of a mixture of alkylbenzenes, in particular fractions of the Solvesso® type,
- chlorinated aliphatic hydrocarbons such as, for example, 1,1,2-trichloroethane, pentachloroethane, 1-iodo-2-methylpropane, 1-chlorohexane and 1-chloro-2-ethylhexane; chlorinated aromatic hydrocarbons and more particularly chlorobenzene and chlorotoluenes,
- ethers and more particularly aliphatic ethers such as butyl ether, isobutyl ether, ethyl hexyl ether, 1-butoxy-2-methoxyethane, 1,1-diethoxybutane, amyl ether, isoamyl ether and dipropoxymethane; aromatic ethers such as phenyl propyl ether and mesityl oxide,
- nitro compounds such as nitropropane and nitrobenzene,
- aliphatic, cycloaliphatic or, preferably, aromatic ketones, methyl ethyl ketone, methyl isobutyl ketone, diisobutyl ketone, methyl n-amyl ketone, methyl isoamyl ketone, cyclohesanone, methylcyclohexanone and diacetone alcohol.

21. Process according to claim 20, **characterized in that** the organic solvent is an aromatic hydrocarbon, preferably cumene or pseudocumene.

22. Process according to one of claims 1 to 21, **characterized in that** the temperature of the dehydration/isomerization reaction is at least 130°C, preferably between 130°C and 180°C and even more preferably between 150°C and 170°C.
